Europäisches Patentamt

European Patent Office

Office européen des brevets

(11) Veröffentlichungsnummer: **0 561 228 A2**

# EUROPÄISCHE PATENTANMELDUNG

(21) Anmeldenummer: **93103442.5**

(51) Int. Cl.⁵: **A61B 1/04**, H04N 9/73

(22) Anmeldetag: **04.03.93**

(30) Priorität: **17.03.92 DE 4208454**

(43) Veröffentlichungstag der Anmeldung:
**22.09.93 Patentblatt 93/38**

(84) Benannte Vertragsstaaten:
**AT CH DE FR GB LI NL**

(71) Anmelder: **Massen, Robert, Prof. Dr.**
**Am Rebberg 29**
**D-78337 Oehningen(DE)**

(72) Erfinder: **Massen, Robert, Prof. Dr.**
**Am Rebberg 29**
**D-78337 Oehningen(DE)**

(74) Vertreter: **Révy von Belvárd, Peter**
**Büchel, von Révy & Partner Patentanwälte,**
**Im Zedernpark Bronschhoferstrasse 31**
**CH-9500 Wil (CH)**

(54) **Verfahren und Anordnung zur Farbbildendoskopie mit Farbtransformation.**

(57) Zur Erzielung von farbtreuen Endoskopiebildern, insbesondere bei medizinischen Untersuchungen, werden im wesentlichen gleichzeitig mit der mit dem zu untersuchenden Flächenbereich (1) auch an der Endoskopspitze vorgesehene Farbreferenzen (4) mit aufgenommen. Durch rechnerischen Vergleich der Farbe dieser Referenzen (4) mit Farbwerten, welche die Referenzen unter kontrollierten Beleuchtungsbedingungen zeigen, wird diejenige Farbraum-Transformationsmatrix berechnet, mit welcher die gemessenen IST-Farbwerte in die SOLL-Farbwerte zurückgerechnet werden können. So werden die Bildpunkte der interessierenden Bildausschnitte vor der Darstellung auf einem Farbmonitor (11) mit dieser Transformationsvorschrift rücktransformiert. Gegebenenfalls können die rücktransformierten Bildpunkte in vorherbestimmte, z.B. zuvor eingelernte, Farbklassen eingeteilt werden und zur Erleichterung der Diagnose als Farbklassenbilod dargestellt werden.

Fig.3

EP 0 561 228 A2

Die Erfindung bezieht auf ein Verfahren gemäß dem Oberbegriff des Anspruches 1 sowie auf eine Anordnung zur Durchführung dieses Verfahrens nach dem Oberbegriff des Anspruches 7.

Bei zahlreichen endoskopischen Untersuchungen in der Medizin und in der Technik werden Farbsensoren, sei es in Form einzelner Diodenarrays oder ganzer Farbkameras verwendet. Üblicherweise wird die zu beobachtende Szene über einen gesonderten optischen Strahlengang, z. B. über ein Lichtleiterbündel, mittels einer Lichtquelle, insbesondere einer Kaltlichtquelle beleuchtet.

Die Farbkamera ist am der Endoskopspitze abgekehrten, beobachtungsseitigen Ausgang des Endoskops, angeschlossen. Ihre Ausgangssignale werden einer Ausgabeeinrichtung, wie insbesondere einem Monitor, zugeführt. Bei neueren Endoskopen kann auch ein Miniatur-Farbbildsensor unmittelbar an der Endoskopspitze angebracht.

Ein bis heute technisch ungenügend gelöstes Problem ist die farbtreue Darstellung der endoskopischen Szenen. Denn durch die künstliche Beleuchtung des jeweils innen befindlichen, zu untersuchenden Flächenbereiches ergibt sich bereits eine Beeinflußung, noch mehr aber durch Reflexe im Inneren, deren Beeinflußung der Farbsignale natürlich nicht vorhersagbar ist und bisher auch nicht bestimmt werden konnte.

Dazu kommt, daß bei medizinischen Untersuchungen die Beleuchtungsoptik infolge sich absetzender Körperflüssigkeit sehr rasch verschmutzen kann. Hierduch ändert sich dann die spektrale Zusammensetzung der Beleuchtung, womit sich über die Zeit Farbintensität, Farbton und Farbsättigung des vom beobachteten Gewebe reflektierten Lichtes verändert. Natürlich können auch bei technischen Untersuchungen Flüssigkeiten vorhanden sein, die das Spektrum verändern mögen.

Zwar verfügen viele endoskopische Farbsysteme über die Möglichkeit eines Weißabgleichs außerhalb der Szene, z.B. über einen getrennten Ausgang der Lichtquelle. Trifft aber im Inneren des Körpers indirekt reflektiertes Licht auf das interessierende Gewebe bzw. den zu untersuchenden Flächenbereich, so ist dieses Licht in seiner spektralen Zusammensetzung nicht gleich dem Weißlicht, welches etwa während eines Weißabgleichs verwendet wurde, so daß es schon aus diesem Grunde zu einer Verfärbung des der beobachteten Farbe des untersuchten Flächenbereiches kommen wird.

Auch sind aus dem Bereiche der graphischen Industrie Verfahren bekannt, um mit Hilfe eines externen Farbsensors die Einstellung eines Farbmonitors zu kalibrieren. Das Einstellen des Farbmonitors leidet aber an einem grundsätzlichen Mangel, denn die eigentlichen Signale (die allenfalls auch zu einer rechnergestützten Auswertung dienen könnten bzw. zur Abspeicherung und Archivierung) werden ja damit nicht verändert, lediglich ihre Darstellung. Zudem sind die Einstellmöglichkeiten eines Monitors begrenzt und gestatten vielleicht eine Veränderung des Farbtones oder der Helligkeit, doch reicht dies ja nicht aus, um den Farbvektor selbst zu verändern, dem bekanntlich mehr als nur diese Determinanten zugeordnet sind.

Aus den obigen Erläuterungen ist ersichtlich, daß hier mehrere Faktoren im Sinne einer Farbverfälschung wirken, und so ist es kein Wunder, daß man sich nachgerade an die Vernachlässigung der Farben bei der Beurteilung des zu untersuchenden Flächenbereiches gewöhnt hat. Im Grunde bedeutet dies aber, daß zwar die Möglichkeiten einer Farbaufnahme gegeben sind, daß für die Diagnose fast ebensogut eine Schwarz-Weiß-Darstellung genügen würde, wenigstens in der Praxis von den Möglichkeiten der Farbdarstellung kaum Nutzen gezogen wird. Jedenfalls werden heute Endoskopieaufnahmen von Medizinern rein qualitativ, d.h. nach Größe und Form, bewertet und eine Interpretation der wechselnden Farbszenen, z.B. im Sinne einer auf Gewebeverfärbungen gestützten Krankheitsdiagnose, ist höchstens mit sehr großer Erfahrung möglich.

Der Erfindung liegt daher die Aufgabe zugrunde ein Verfahren der eingangs genannten Art so auszubilden, daß wirklich farbtreue Endoskopiebilder gewonnen werden können. Erfindungsgemäß gelingt die Lösung dieser Aufgabe durch die kennzeichnenden Merkmale des Anspruches 1.

Dadurch, daß die Farbreferenz unter denselben Beleuchtungsbedingungen gewonnen wird, wie das Bild des zu untersuchenden Flächenbereiches selbst, ist gesichert, daß eine richtige Anpassung der Farbsignale erreicht werden kann. Dadurch, daß die Anpassung mit Hilfe einer Transformationsvorschrift für die Farbvektoren durchgeführt wird, ist sichergestellt, daß nicht einzelne Determinanten derselben verändert werden, sondern alle diese Vektoren bestimmenden Einflußgrößen, d.h. eben Farbintensität, Farbton und Farbsättigung. Dabei wirken diesen beiden Merkmale auch dahingehend zusammen, daß auch eine Veränderung der Beleuchtungsverhältnisse im Laufe der Untersuchung, z.B. durch Verschmutzung der Optik od.dgl., jederzeit ausgeglichen werden können.

Durch das Merkmal c) des Anspruches 1 wird schließlich gesichert, daß die der Ausgabeeinrichtung zuzuführenden Signale selbst im Sinne einer getreuen Farbwiedergabe korrigiert werden, was nicht nur die farbtreue Ausgabe auf einem Monitor sichert, sondern auch die Möglichkeit einer Ausgabe an eine rechnergestützte Diagnoseeinrichtung, die mit Hilfe der nun richtigen Farbinformation zu besseren Ergebnissen gelangen kann. Schließlich ist auch noch die Möglichkeit der Ausgabe an eine Speichereinrichtung, insbesondere auf einem nicht-flüchtigen Speicher, wie Magnetband oder -platte, CD-ROM u.dgl. zu Archivierungszwecken, z.B. für einen späteren Vergleich, unter diesen Umständen sinnvoll. Dabei ist es im Rahmen der Erfindung durchaus möglich, alle Bildpunkte mit einer einzigen, der durchschnittlichen Veränderung der Farbvektoren entsprechenden Transformationsvorschrift umzurechnen. Es könnte aber auch der Fall sein, dass einzelne Abschnitte des Farbbildes durch unterschiedliche Gegebenheiten (z.B. Reflexion von Flächen stark unterschiedlicher Farbe) mit unterschiedlichen, entsprechend adaptierten Transformationsvorschriften umgerechnet werden. Schliesslich ist zu bedenken, dass für den Vergleich von SOLL- und IST-Farbwerten die Werte der einzelnen Bildpunkte herangezogen werden können (alternativ nur die Bildpunkte ausgewählter Flächenbereiche), so dass es theoretisch möglich wäre, jeden Bildpunkt mit einer gesonderten Transformationsvorschrift umzurechnen.

Weitere Einzelheiten der Erfindung ergeben sich an Hand der nachfolgenden Beschreibung von in der Zeichnung schematisch dargestellten Ausführungsbeispielen. Es zeigen:

Fig. 1    einen Querschnitt durch eine Körperhöhlung die von einem Endoskop entsprechend dem Stande der Technik untersucht wird;

Fig. 2    eine der Fig. 1 ähnliche Darstellung mit einem erfindungsgemäß ausgebildeten Endoskop, wozu die

Fig. 3    das Endsokop mit der angeschlossenen Schaltung und einem Monitor veranschaulicht, wogegen die

Fig. 4    eine Ausführungsvariante zeigt.

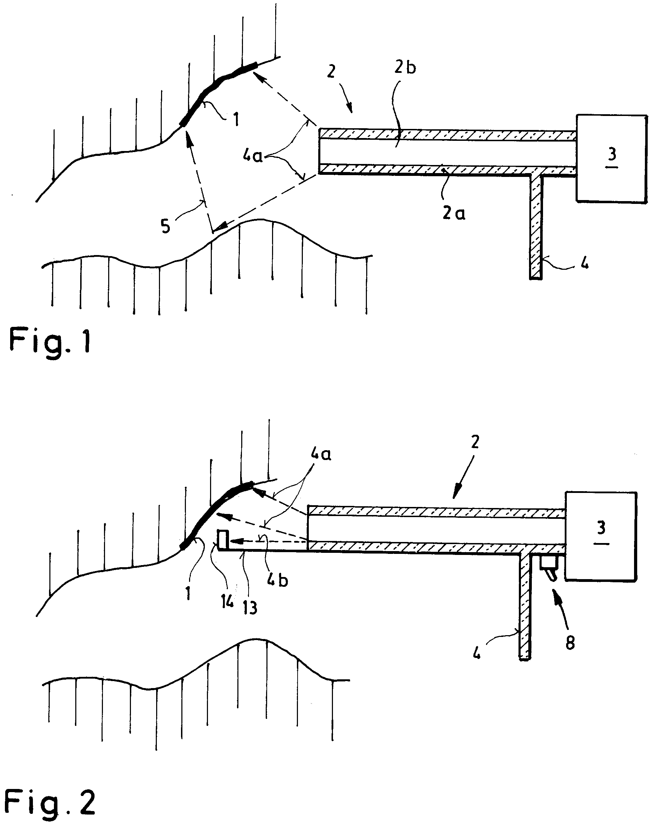

An Hand der Fig. 1 soll die der Erfindung zugrundeliegende Problematik aufgezeigt werden. Ein zu untersuchender Flächenbereich 1 eines Gewebes in einer Körperhöhlung (z.B. einem Darmstück), wird von einem Endoskop 2 untersucht und über einen äußeren, einen zentralen Aufnahmestrahlengang 2b umgebenden, lediglich schematisch angedeuteten Strahlengang 2a mit Hilfe eines Kaltlichtquellenanschlusses 4 mit einem divergierenden Beleuchtungsstrahl 4a beleuchtet. Es ist ersichtlich, daß der Beleuchtungsstrahl 4a auch von anderen Teilen des Gewebes, außerhalb des Flächenbereiches 1, reflektiert wird, wobei sich Reflexionsstrahlengänge 5 ergeben, die nach teilweiser Absorption eines von der Reflexionsstelle abhängenden Spektrums auf den zu untersuchenden Flächenbereich 1 auftreffen und so dessen Beleuchtungsspektrum gegenüber dem des Strahles 4a verändern. Schon damit verändert sich die Farbe des beobachteten Gewebes 1 in einer kaum vorhersehbaren Weise gegenüber der direkten Beleuchtung durch den Strahl 4a, so daß sich am Endoskopausgang 3, wo ein entsprechender Farbsensor, insbesondere eine Farbkamera, angebracht ist, an sich verfälschte Farbsignale ergeben.

Hinzu tritt eine mögliche Veränderung des Spektrums des Strahles 4a selbst, einerseits durch mögliche Veränderungen des Spektrums der Beleuchtungsquelle 4 über die Zeit (was noch am ehesten durch entsprechende Messungen, beispielsweise im Strahlengang 4a beherrschbar wäre), anderseits aber durch mögliche Verschmutzung der Beleuchtungsoptik des Endoskopes 2, z.B. durch Niederschlag von Körpersäften, deren Zusammensetzung und Ausmaß natürlich ebensowenig vorhersehbar ist.

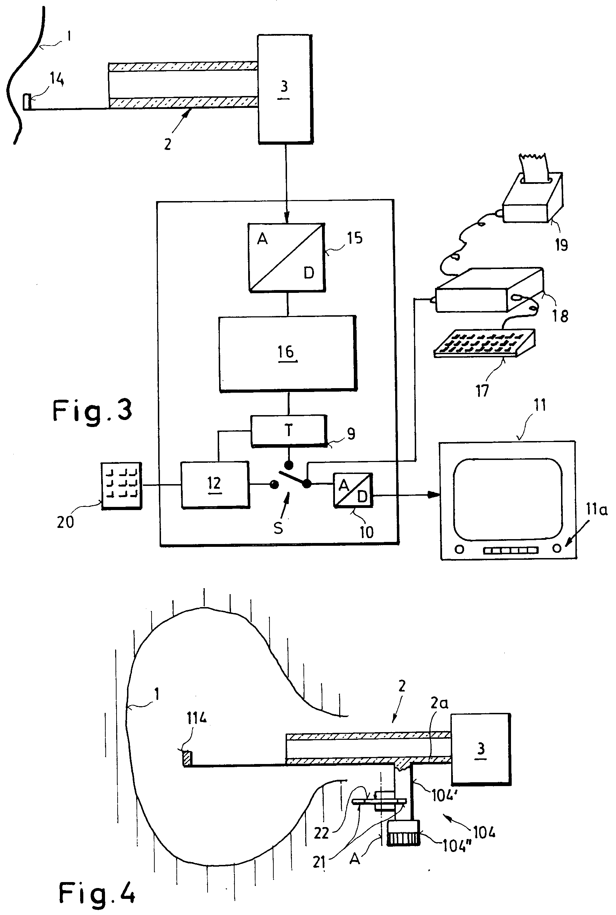

Diese Effekte, insbesondere durch Verschmutzung der Beleuchtungsoptik und durch Beleuchtung über reflektiertes Licht 5, führen dazu, daß sich der Farbeindruck an einer Ausgabeeinrichtung, wie einem Monitor 11 (vgl. Fig. 3), laufend ändert, Eine absolute Farbanalyse oder Farbinterpretation ist daher nicht mehr möglich, selbst wenn vor der Untersuchung ein Weißabgleich durchgeführt wurde. Noch weniger wäre eine, an sich bekannte, automatische Echtzeit-Farbklassifikation (vgl. MASSEN, R.: "Colour and Shape Classifikation with competing paradigms; Neural Networks versus Trainable Table Classifiers" ECO 3 Int. Congress Optical Science, Den Haag, March 1990) einzusetzen, die eine rechnergestützte Diagnose durch direkte Klassifikation des Gewebes in kranke und gesunde Abschnitte ermöglichen würde.

Diese Probleme werden nun mit einer Anordnung gemäß den Fig. 2 und 3 gelöst. Wird nämlich eine Szene einer homogenen SOLL-Farbe

**Fsoll** = [Rs, Gs, Bs]

mit einem Licht (Strahlen 4a, 5) beleuchtet, dessen Spektrum sich gegenüber dem bei einer SOLL-Messung verwendeten Referenzspektrum verändert, so erhält dieses Objekt die neue IST-Farbe

**Fist** = [Ri, Gi, Bi].

Hierbei gilt die Transformationsvorschrift
bzw.

$$\textbf{Fist = Fsoll x T} \qquad [1]$$

$$\textbf{Fsoll = Fist x } \textbf{T}^{-1}$$

wobei die fett gedruckten Buchstaben, wie auch im folgenden, Vektoren und Matrizen bezeichnen, dagegen Buchstaben im Normaldruck die Komponenten der Vektoren und die Koeffizienten der Matrizen, und T eine 3 x 4 Transformationsmatrix zur Überführung des Farbvektors **Fsoll** in den Farbvektor **Fist** und $\textbf{T}^{-1}$ die inverse Matrix ist.

Die 3x4 = zwölf Komponenten dieser Matrix bestimmen die Drehung, Translation und die Skalierung (Dehnung bzw. Stauchung) des SOLL-Farbraumes für den jeweiligen Farbvektor, d.h. diejenige affine Transformation, welcher die SOLL-Farbe **Fsoll** dadurch unterworfen wird, daß sie mit einem Licht beleuchtet wird, dessen Spektrum sich gegenüber einem Referenzspektrum verändert hat. Es sei erwähnt, daß eine solche Transformationsvorschrift sich unabhängig von der Art eines betrachteten Farbraumes ergibt, sei es der RGB-Farbraum (unter Bedachtnahme auf die Anteile der drei Grundfarben), der IHS-Farbraum (mit Berücksichtigung von Intensität, Farbton oder Hue und Sättigung), der Lab-, der xyz- oder ein ähnlicher Farbraum.

Die unbekannten Koeffizienten der Transformationsmatrix **T** lassen sich durch Auflösen der Matrizengleichung nach **T** an Hand der Vermessungen der Farbreferenzen bestimmen. Dies führt auf ein System von 12 linearen Gleichungen, deren Lösung die Koeffizienten der Matrix **T** darstellen. Hierzu ist es erforderlich, mindestens n = 4 unterschiedliche bekannte Farbreferenzen $\textbf{Fref}_i$ einmal unter kontrollierten SOLL-Bedingungen, d.h. mittels einer Weißlichtquelle definierten Referenzspektrums, zu bestimmen. 4 Farbreferenzen stellen dann 4 mal 3 = 12 Farbkomponenten pro Farbe und damit 12 bekannte Werte dar. Durch Ausmessung der entsprechenden Farbreferenzen $\textbf{F'ref}_i$ im Inneren des Körpers (oder eines technischen Hohlraumes) unter den dort herrschenden Beleuchtungsbedingungen werden die IST-Farbwerte dieser Referenzen bestimmt. Die Koeffizienten der Matrix **T** lassen sich dann durch Auflösen der Matrixgleichung

$$\textbf{F'ref}_i = \textbf{Fref}_i \times \textbf{T} \; ; \; \textbf{i} \geq 4 \qquad [2]$$

mit bekannten mathematischen Verfahren ermitteln. Werden mehr als die minimale Anzahl von 4 benötigten Farbreferenzen verwendet, so ist das Gleichsystem [2] überbestimmt und kann vorteilhaft mit den Verfahren des kleinsten Fehlerquadrates so gelöst werden, daß robustere Mittelwerte für die Matrixkoëffizienten erhalten werden. Diese Verfahren der Matrixmathematik sind dem Fachmanne an sich bekannt und brauchen daher hier nicht näher behandelt werden.

Ausgehend von diesen Basisüberlegungen wird gemäß Fig. 2 bei der Untersuchung der Gewebefläche 1 gleichzeitig eine an der Spitze des Endoskopes 2 mittels eines lediglich schematisch angedeuteten Trägers 13 gehaltene Referenzfläche 14 mit betrachtet, die so in unmittelbarer Nähe des zu untersuchenden Flächenbereiches 1 angeordnet werden kann und über einen Teilstrahl 4b der Beleuchtungseinrichtung 4 beleuchtet wird. Der Träger 13 kann ein für die Anbringung verschiedener Behelfe normalerweise an einem Endoskop 2 vorgesehener Träger sein. Ebenso ist es an sich bekannt, im Bereiche des Endoskopausganges 3 eine, lediglich schematisch angedeutete, Betätigungseinrichtung 8 vorzusehen, mit deren Hilfe der Träger 13 willkürlich und von Hand aus bewegt werden kann, insbesondere auf die Fläche 1 zu und von dieser weg bzw. sogar ganz aus dem Endoskop 2 herausgezogen werden kann. Möglich wäre gegebenenfalls auch, den Träger 13 und seine Betätigungseinrichtung 8 derart auszubilden, daß eine seitliche Bewegung quer zur optischen Achse durchführbar ist, etwa um die Referenzfläche 14 in die Nähe eines seitlich gelegenen Flächenbereiches 1 zu bringen.

Die Referenzfläche 14 dient dazu, eine Ableitung einer Reihe von (mindestens 4) Farbreferenzen von dieser Referenzfläche zu ermöglichen, und dies geschieht im einfachsten Falle dadurch, daß an der Referenzfläche 14 die Farbreferenzen selbst als Referenzfläche 14 vorgesehen sind. Dabei kann die Gestaltung der Farbreferenzen an der Referenzfläche 14 verschieden, beispielsweise streifen-, kreis- oder ringförmig sein. Wesentlich ist, daß dadurch einerseits das Blickfeld auf die Fläche 1 nicht zu sehr eingeengt wird, anderseits aber die Plazierung so erfolgt, daß die Referenzfläche unter denselben Beleuchtungsverhältnissen wie die zu untersuchende Fläche 1 steht. Eine Alternative zu dieser Möglichkeit der unmittelbaren Anbringung der Farbreferenzen an der Referenzfläche 14 wird später an Hand der Fig. 4 erläutert.

Die Messung der Farbreferenzen $F'ref_i$ im Körper bzw. der jeweiligen Höhlung und der Vergleich mit den außerhalb des Körpers unter kontrollierten Bedingungen vorherbestimmten Farbreferenzen $Fref_i$ ermöglicht nach Auflösung der obigen Gleichung [2] die Bestimmung der Transformationsmatrix $T$, welche die Soll-Farben in die Ist-Farben überführt. Die inverse Transformationsmatrix $T^{-1}$ wird auf alle Bildpunkte mit der Farbe $Fist_k$ des eigentlich interessierenden Gewebebildes angewendet, wobei der Buchstabe $k$ für alle Bildpunkte des Gewebebildes steht

$$Fsoll_k = T^{-1} \times Fist_k \qquad [3]$$

und das Gewebebild erst nach dieser Transformation zur Ausgabe, sei es in einen Rechner zur diagnostischen Auswertung, sei es in einen Speicher, an einen Drucker und/oder an einen Monitor 11 (vgl. Fig. 3), gebracht wird.

Die Transformation findet - als rechnerischer Vorgang - gemäß Fig. 3 mit Hilfe eines Transformationsrechners 9 statt, dem eine Speichereinrichtung 16 zugeordnet ist. Diese Speichereinrichtung 16 kann entweder als einheitlicher Speicher mit mehreren Speicherabschnitten ausgebildet sein oder mehrere gesonderte Speicher enthalten, um einerseits das vom Endoskop 2 aufgenommene jeweilige Bild (Bildspeicherabschnitt) zu speichern, anderseits die zuvor unter kontrollierten Bedingungen aufgenommenen SOLL-Farben, mit denen die IST-Farben des Bildes verglichen werden soll (Farbspeicherabschnitt) und gegebenenfalls auch das Transformationsprogramm für den Rechner 9 (soferne dies nicht im Rechner 9 selbst enthalten ist), welche letzteres praktisch die obigen Gleichungen [1] bis [3] samt etwaigen Matrixgleichungen bzw. die für die Methode der kleinsten Fehlerquadrate erforderlichen Rechenprogramme umfaßt. In jedem Falle wird hierdurch der Farbraum auf Grund des Vergleiches zwischen SOLL-Farben und IST-Farben so rücktransformiert, daß die Wirkung der veränderten Beleuchtung, etwa durch Verschmutzung des Beleuchtungskanales bzw. durch indirekte Reflexion (vgl. den Strahl 5 in Fig. 1) rückgängig gemacht wird. Die an die Ausgabeeinrichtung bzw. den Monitor 11 gebrachten Farbsignale $Fsoll_k$ des zu untersuchenden Flächenbereiches 1 eines Gewebes, eines technischen Gerätes oder in einer anderen Höhlung entsprechen damit den absolut treuen Farben, welche unter idealen Beleuchtungsbedingungen erzielt würden.

Damit nun diese Farbsignale nicht bei der Darstellung durch den Monitor 11 selbst eine Verfälschung erfahren, ist es vorteilhaft, wenn dieser Farbmonitor nach den aus der graphischen Industrie an sich bekannten Verfahren vor der endoskopischen Untersuchung kalibriert wird. Dies kann vorzugsweise so erfolgen, daß zur absoluten Kalibrierung des Farbmonitors 11 auf demselben die Farbreferenzen dargestellt werden, die über das Endoskop im wesentlichen beleuchtungsfrei, d.h. unter Abschaltung der Lichtquelle 4 (gegebenenfalls auch nur gedimmt) aufgenommen werden, und daß aus dem Vergleiche der Farbe der Farbreferenzen und der IST-Farbe der auf dem Farbmonitor dargestellten Farbreferenzen die Einstellparameter für die Kalibrierung berechnet werden. Hierauf kann die Einstellung des Farbmonitors 11 mittels der an ihm üblicherweise vorgesehenen Einstellelemente 11a kalibriert werden.

Fig. 3 zeigt oben wieder den in Fig. 2 in größerem Maßstabe dargestellten Teil mit dem Endoskop 2 und der Referenzfläche 14 zur Ableitung von Referenzfarbsignalen. Der Ausgang 3 des Endoskops 2 ist zweckmäßig an einen Analog/Digital-Wandler 15 angeschlossen, um nach Digitalisierung in den schon erwähnten Bildabschnitt der Speichereinrichtung 16 eingelesen zu werden. Diese Speichereinrichtung ist an den Bildrechner 9 angeschlossen, Der Rechner 9 mißt die Farbvektoren der ihm von der Speichereinrichtung 16 gelieferten Farbreferenzsignale, die einerseits von der Referenzfläche 14 abgeleitet sind (IST-Farbsignal) und vergleicht diese mit den von einem SOLL-Farbenabschnitt der Speichereinrichtung 16 gelieferten SOLL-Farbsignalen, die unter kontrollierten Bedingungen vor der Untersuchung in die Speichereinrichtung 18 eingelesen worden waren und berechnet nach der Transformationsgleichung [2] die Koeffizienten der Transformationsmatrix $T$.

Das Einlesen der n Referenzfarben in die Speichereinrichtung 16 (oder einen im Rechner 9 enthaltenen Speicher) kann durch einen "Lernvorgang" vor der eigentlichen Untersuchung unter idealen Beleuchtungsbedingungen, z.B. in einem abgeschirmten Dunkelraum erfolgen, wo die Farben aufgenommen, ihre Vektoren, z.B. über den Rechner 9 oder einen äquivalenten Rechner, vermessen und in der Speichereinrichtung 16 bzw. dem Bildrechner 9 selbst abgespeichert werden.

Der Rechner 9 kann einen Speicher für das transformierte Bild besitzen, in den die Daten nach der Ermittlung der Transformationskoëffizienten und Umrechnung der Vektoren eingelesen werden. Alternativ wird das transformierte Bild aus dem Rechner 9 mehr oder minder unmittelbar an wenigstens eine der Ausgabeeinrichtungen 11, 18, 19 ausgegeben. Zu diesem Zwecke wird das vom Endoskop 2 bzw. dem Bildspeicher 16 erhaltene Bild mit Hilfe der ermittelten Transformationskoëffizienten entsprechend Gleichung [3] transformiert, wobei der Rechner mit verschiedenen Ausgabeeinrichtungen verbunden sein kann, beispielsweise einem über ein Tastenfeld 17 bedienbaren Diagnosecomputer 18 sowie einen Drucker 19, der gegebenenfalls ein Videodrucker sein kann, um die beobachteten Szenen, allenfalls nach Auswertung durch den Computer 18, zu Papier zu bringen.

Alternativ werden die dem transformierten Bild entsprechenden Signale an einen an den Rechner 9 angeschlossenen Farbklassifikator 12 abgegeben und dort Bildpunkt für Bildpunkt in vorbestimmte Farbklassen eingeordnet, die beispielsweise zuvor im Farbklassifikator 12 abgespeichert wurden. Sodann kann die Ausgabe über den Farbklassifikator 12 nach Umschalten eines Wechselschalters S , gegebenenfalls über einen Digital/Analog-Wandler 10, als Farbklassenbild erfolgen. Zur Erläuterung dieses nun schon an sich bekannten Verfahrens sei auf die oben genannte Literaturstelle von MASSEN hingewiesen.

Durch die Farbklassifikation ist es möglich, jedem Bildpunkt die Farbe einer entsprechenden Farbklasse zuzuordnen, die an sich eine dem Ideal entsprechende "Standardfarbe" sein kann, die aber nach Belieben gegebenenfalls über eine Eingabeeinrichtung 20 in eine Falschfarbe umgewandelt werden kann. So ist es durch entsprechende Falschfarbendarstellung möglich, beispielsweise als krank oder verdächtig klassifizierte Gewebeausschnitte dem menschlichen Auge des Diagnostikers besonders prägnant darzustellen, was die Diagnosesicherheit des Arztes erhöht. Dabei kann die Diagnosesicherheit verbessert werden, wenn das so erhaltene Farbklassenbild (allenfalls aber auch das Bild der retransformierten Echtfarben) rechnerisch ausgewertet wird, also beispielsweise Farbklassifikator 12 und Computer 9 über einen Bus zum gegenseitigen Informationsaustausch miteinander verbunden sind, wobei dem Computer 9, beispielsweise aus der Speichereinrichtung 16, ein Diagnoseprogramm zugeordnet ist, das aus auffälligen Verfärbungen auf bestimmte Krankheiten (oder Korrosionsmängel an einem technischen Gegenstand) schließt. Gegebenenfalls kann es aber auch genügen, auf diese auffälligen Farben hinzuweisen oder ein entsprechendes Symobol, eine Ziffer od.dgl., an- bzw. auszugeben. Anderseits kann es auch nützlich sein, über ein solches Symbol den Unterschied zwischen der wahren Farbe und der Farbklasse anzuzeigen.

Gemäß einer Weiterbildung der Erfindung besitzt der Programmabschnitt des dem Bildrechner 9 zugeordneten Speichers auch ein Programm zur Ermittlung quantitativer Maße, wie die relative Größe der einzelnen Farben bzw. Farbklassen, Form- und Texturmerkmale sowie die prozentuale Abweichung zu den abgespeicherten bzw. trainierten Farben oder Farbklassen, um so zusätzliche Angaben zur Diagnosehilfe zu errechnen und den Ausgabeeinrichtungen 11, bzw. 18, 19, z.B. auch in Zahlenwerten und/oder Symbolen, zuzuführen.

Die durch die Erfindung erzielbaren Vorteile liegen auf der Hand. Im übrigen ist es ohne weiteres möglich, bestehende Endoskope mit einer Referenzfläche 14 mit Farbreferenzen nachzurüsten, welche durch den im allgemeinen vorhandenen Operationskanal des Endoskopes 2 (nicht dargestellt), d.i. jener Kanal, durch den der Arzt chirurgische Instrumente einzuführen vermag, eingeführt und in das Blickfeld der Kamera am Ausgange 3 gebracht werden können. Selbstverständlich muß dann die an Hand der Fig. 3 beschriebene Auswerteschaltung an die Kamera 3 angeschlossen werden.

Im Falle der Fig. 4 ist die Referenzfläche 114 mit einer Weißreferenz versehen, die zur Ableitung der Farbrefernzen über eine Beleuchtungseinrichtung 104 farbig bestrahlt werden kann. Zu diesem Zwecke ist die Beleuchtungseinrichtung mit einem Beleuchtungsschacht 104' versehen, der an das Endoskop über eine an sich bekannte und deshalb hier im einzelnen nicht gezeigte Einspiegelungseinrichtung bzw. über Lichtleiter das Licht einer in einem Lampengehäuse 104'' untergebrachten Lichtquelle in den Beleuchtungsstrahlengang 2a einbringt. In den Strahlengang des Beleuchtungsschachtes 104' ist gewünschtenfalls jeweils eines von mehreren Filtern 21 einschwenkbar, die über den Umfang einer um eine Achse A drehbaren Scheibe 22 angeordnet sind. Auf diese Weise kann die Ermittlung der Referenzfarben durch aufeinanderfolgendes Einschwenken der Filter 21 in den Schacht 104' erfolgen, so daß die Weißreferenz 114 der Reihe nach mit standardisierten Farben beleuchtet wird. Die dabei über den Farbsensor 3 gewonnen Signale werden dann ebenso ausgewertet, wie oben beschrieben.

Im vorliegenden Falle spielt die Filterscheibe 22 die Rolle einer Schaltvorrichtung zum Umschalten der Farben. Allerdings wäre es ebenso denkbar, mehrere Lichtquellen für Licht unterschiedlichen Spektrums vorzusehen und diese Lichtquellen nacheinander über eine elektrische Schalteinrichtung ein- bzw. auszuschalten.

Es versteht sich, daß die zuletzt genannte Ausbildung zwar im Rahmen der Erfindung möglich ist, die Anbringung der Farbreferenzen an der Fläche 4 aber bevorzugt ist. Anderseits ist dieses Verfahren leicht auch dazu verwendbar, den Monitor 11 zu kalibrieren. Auch wäre es denkbar, zusätzlich zur Farbreferenz auch eine Weißreferenz an der Referenzfläche vorzusehen.

## Patentansprüche

1. Verfahren zur Gewinnung von Farb-Endoskopiebildern, insbesondere für medizinische und technische Untersuchungen, mit Hilfe eines lichtelektrischen Farbbildsensors, dessen Signale einer Ausgabeeeinrichtung zugeführt werden, **dadurch gekennzeichnet,** daß zur Gewinnung farbtreuer Endoskopiebilder

a) mit dem zu untersuchenden Flächenbereich eine Reihe von auch Farbreferenzen mit exakt vorherbestimmten SOLL-Farbenwerten dem Farbbildsensor zugeführt werden,

b) daß durch Vergleich der IST-Farben dieser Referenzen mit deren SOLL-Farbenwerten eine Transformationsvorschrift für die Farbvektoren zur Überführung der IST-Farben des zu untersuchenden Flächenbereiches in die der Abweichung zwischen IST- und SOLL-Werten der Farbreferenzen entsprechenden Farbwerte des zu untersuchenden Flächenbereiches ermittelt wird, und

c) daß die Farben aller Bildpunkte des zu untersuchenden Flächenbereiches vor der Zuführung an die Ausgabeeinrichtung mit Hilfe der so gewonnen Transformationsvorschrift umgerechnet werden.

2. Verfahren nach Anspruch 1, dadurch gekennzeichnet, daß

a) die transformierten Bildpunkte des zu untersuchenden Flächenbereiches nach erfolgter Transformation jeweils in eine von mehreren vorherbestimmten Farbklassen klassifiziert werden, und

b) daß der Ausgabeeinrichtung jeweils das dem Farbklassenbild entsprechende Signal zugeführt wird.

3. Verfahren nach Anspruch 2, dadurch gekennzeichnet, daß wenigstens eine der folgenden Maßnahmen durchgeführt wird:

a) nach Einordnen der transformierten Bildpunkte jeweils in eine Farbklasse wird der jeweiligen Farbklasse ein Code zugeordnet und der Ausgabeeinrichtung in Abhängigkeit vom jeweiligen Code ein Falschfarbenbild zugeführt;

b) es wird die Abweichung der rücktransformierten IST-Farben zu vorherbestimmten SOLL-Farbklassen berechnet und ein entsprechendes Signal der Ausgabeeinrichtung zugeführt.

4. Verfahren nach Anspruch 1 oder 2, dadurch gekennzeichnet, daß wenigstens eine der folgenden Massnahmen durchgeführt wird:

a) es wird rechnerisch eine Auswertung der geometrischen Strukturen, wie Form und/oder Größe, und/oder der Texturen, insbesondere im Farbklassenbild, zur Ableitung einer entsprechenden Information vorgenommen und diese der Ausgabeeinrichtung zugeführt;

b) das jeweilige Bild, insbesondere das Farbklassenbild, wird zum Erhalt eines ihm entsprechenden, die Diagnose unterstützenden Symbols rechnerisch ausgewertet und ein dem Symbol entsprechendes Signal der Ausgabeeinrichtung zugeführt.

5. Verfahren nach einem der vorhergehenden Ansprüche, dadurch gekennzeichnet, daß die Ausgabeeinrichtung wenigstens einen Farbmonitor aufweist, der zur Untersuchung des Flächenbereiches farbkalibriert wird, und daß vorzugsweise zur absoluten Kalibrierung dieses Farbmonitors auf demselben die Farbreferenzen dargestellt werden, die über das Endoskop im wesentlichen beleuchtungsfrei aufgenommen werden, und daß aus dem Vergleiche der Farbe der Farbreferenzen und der IST-Farbe der auf dem Farbmonitor dargestellten Farbreferenzen die Einstellparameter für die Kalibrierung berechnet werden.

6. Verfahren nach einem der vorhergehenden Ansprüche, dadurch gekennzeichnet, daß zum Erhalt der Farbreferenzen zunächst gleichzeitig mit dem zu untersuchenden Flächenbereich eine Weißreferenz dem Farbbildsensor zugeführt wird, daß diese Weißreferenz durch mehrere farbige Lichtquellen vorbestimmter spektraler Verteilung beleuchtet werden, und daß aus den Bildern der Weißreferenz unter diesen Beleuchtungen und der bekannten, vorbestimmten spektralen Verteilung der Beleuchtungen diejenige Transformationsvorschrift für die Farbvektoren ermittelt wird, welche die IST-Weißfarbe in die SOLL-Weißfarbe überführt.

7. Anordnung zur Durchführung des Verfahrens nach einem der vorhergehenden Ansprüche, mit einem an eine Endoskopeinrichtung (2) mit einer an einen zu untersuchenden Flächenbereich (1) heranzuführenden Endoskopspitze angeschlossenen lichtelektrischen Farbbildsensor (3), dessen Signale einer Ausgabeeinrichtung (11, 18, 19) zuführbar sind, **dadurch gekennzeichnet**, daß an der Endoskopspitze eine Referenzfläche (14; 114) zur Abbildung auf dem Farbsensor (3) mit dem zu untersuchenden Flächenbereich (1) vorgesehen ist, daß eine Einrichtung (9; 104) zur Ableitung einer Reihe von Farbreferenzen von dieser Referenzfläche (14; 114) vorgesehen ist, daß der Ausgang des Farbsensors (3) mit einer einem Bildrechner (9) zugeordneten IST-Farbabschnitt einer Speichereinrichtung (16) verbunden ist, wobei die Farbe der Farbreferenzen mit Hilfe des Bildrechners (9) bestimmbar und mit in einem SOLL-Farbenabschnitt der Speichereinrichtung (16) abgespeicherten SOLL-Farbreferenzen vergleichbar ist, daß der Rechner (9) mittels eines in einem Programmabschnitt der Speichereinrichtung (16) Rechenprogrammes zur Berechnung einer Transformationsvorschrift auf Grund des Vergleiches von IST- und SOLL-Farbe und zur Umrechnung der Bildpunkte des gespeicherten Bildes entsprechend der errechneten Transformationsvorschrift ausgebildet ist.

**8.** Anordnung nach Anspruch 7, dadurch gekennzeichnet, daß die Referenzfläche (14) selbst eine Reihe von Farbreferenzen aufweist und die Einrichtung zur Ableitung einer Reihe von Farbreferenzen bildet,

**9.** Anordnung nach Anspruch 7 oder 8, dadurch gekennzeichnet, daß die Referenzfläche (14; 114) auf einem mit Hilfe einer manuell von einer Bedienungsperson betätigbaren Betätigungseinrichtung (8) beweglichen Träger (13) angeordnet ist, daß der Träger (13) vorzugsweise wahlweise in das Endoskop (2) ein- bzw. ausfahrbar ist, und daß er insbesondere über einen Operationskanal des Endoskops (2) bis ins Blickfeld des Farbsensors (3) einschiebbar ist.

**10.** Anordnung nach einem der Ansprüche 7 bis 9, dadurch gekennzeichnet, daß die Referenzfläche (114) einen Bereich mit einer Weißreferenz aufweist, und daß eine Farbbeleuchtungseinrichtung (104) für mehrere vorbestimmte Farben einer vorbestimmten spektralen Verteilung zum, insbesondere über eine Schalteinrichtung (22) aufeinanderfolgenden, Beleuchten des Weißreferenzbereiches (114) vorgesehen ist, wobei der Rechner (9) zum Berechnen derjenigen Transformationsvorschrift für die Farbvektoren aus den Bildern der Weißreferenz unter diesen Beleuchtungen und der bekannten, vorbestimmten spektralen Verteilung der Beleuchtungen ausgebildet ist, welche die IST-Weißfarbe in die SOLL-Weißfarbe überführt.

Fig. 1

Fig. 2

Fig.3

Fig.4